# EUROPÄISCHE PATENTSCHRIFT

(11) **EP 0 793 977 B1**
(45) Veröffentlichungstag und Bekanntmachung des Hinweises auf die Patenterteilung: **20.08.2003**
(21) Anmeldenummer: 97250054.0
(22) Anmeldetag: 04.03.1997
(51) Int. Cl.: A61N 1/368, A61N 1/39, A61M 5/172, A61N 1/365

(54) **Therapiegerät**
Therapy device
Appareil de thérapie

(30) Priorität: 04.03.1996 DE 19609409
(43) Veröffentlichungstag der Anmeldung: 10.09.1997
(73) Patentinhaber: BIOTRONIK Mess- und Therapiegeräte GmbH & Co Ingenieurbüro Berlin, 12359 Berlin (DE)
(72) Erfinder: Thong, Tran, Lake Oswego, Oregon 97035 (US); Digby, Dennis, Lake Oswego, Oregon 97035 (US); Schaldach, Max, Prof. Dr.-Ing., 91054 Erlangen (DE)
(74) Vertreter: Eisenführ, Speiser & Partner

(56) Entgegenhaltungen:
- EP-A- 0 841 074
- WO-A-89/06990
- WO-A-96/02185
- DE-C- 3 732 640
- US-A- 5 330 505
- US-A- 5 487 752

## Beschreibung

Die Erfindung betrifft ein Therapiegerät der im Oberbegriff des Anspruchs 1 angegebenen Art.

Selbsttätig arbeitende medizinische Therapiegeräte sind insbesondere und seit längerem als implantierbare Herzschrittmacher zur Behandlung von bradykarden und/oder tachykarden Herzrhythmusstörungen, aber auch zunehmend als automatische Defibrillatoren bzw. Kardioverter, als kombinierte Schrittmacher/Kardioverter oder als implantierte Medikamentendosierpumpen o.ä. - allgemein bekannt und im alltäglichen medizinischen Einsatz.

Modernere Geräte dieser Art sind mikroprozessorgesteuert und bieten die Möglichkeit der individuellen, auf ein konkretes Krankheitsbild zugeschnittenen Programmierung einer von einer Mehrzahl vorinstallierter Betriebsweisen, mit der eine vorgegebene Therapie realisiert wird, und zugehöriger Betriebsparameter (im weiteren auch als Therapiegrößen bezeichnet, soweit sie therapeutisch relevant sind).

Bekannt sind -etwa aus DE 3 732 640 oder US 5,330,505- insbesondere auch gattungsgemäße Geräte, die mit einem oder mehreren Fühler(n) zur Aufnahme von diagnostisch relevanten Signalen im Körper des Patienten und zugehörigen Signalaufbereitungs- und -verarbeitungseinrichtungen sowie einer Auswertungs- und Steuereinheit ausgerüstet sind, die gemäß einem im Gerät gespeicherten Algorithmus in Abhängigkeit vom Wert bzw. den Werten der aufgenommenen Größe(n) aus der Menge der programmierten Betriebsparameter bzw. Therapiegrößen jeweils einen aktuellen Parameter bzw. Parametersatz berechnet. Hierzu zählen etwa Herzschrittmacher, bei denen die Stimulationsrate in Abhängigkeit von der körperlichen Aktivität des Trägers gesteuert wird. Weiterhin sind Therapiegeräte bekannt, die zu einer automatischen Aktivierung oder - insbesondere ebenfalls vorprogrammierten - Umschaltung aus einer Betriebsart in eine andere in Abhängigkeit vom Wert einer im Körper des Patienten erfaßten Größe ausgebildet sind. Hierzu zählen die bekannten Bedarfs-Herzschrittmacher oder automatischen Defibrillatoren und die in neuerer Zeit entwickelten Kombinationsgeräte.

Diese Geräte werden bei der Implantation entsprechend dem Krankheitsbild und ggfs. den Lebensbedingungen (beispielsweise der durchschnittlichen körperlichen Aktivität) des Patienten programmiert, wobei auch der anzuwendende Algorithmus zur Bestimmung der Therapie bzw. Therapiegröße(n) in Abhängigkeit vom Wert der im Körper erfaßten Größe(n) festgelegt wird. Bei den in bestimmten Abständen erfolgenden Nachsorgeuntersuchungen können durch eine Umprogrammierung sowohl der Betriebsart- und -paramatersatz als auch - falls das Therapiegerät über mehrere gespeicherte Algorithmen verfügt - der anzuwendende Steueralgorithmus geändert werden

Jedoch ist in den Betriebsphasen zwischen den Untersuchungen über den aktivierten Algorithmus eindeutig festgelegt, welche Therapie bzw. Therapiegröße das Gerät bei einem bestimmten Wert bzw. bestimmten Werten der im (oder am) Körper erfaßten Meßgröße liefert - beispielsweise, welche Stimulationsfrequenz eines ratenadaptiven Schrittmachers einem bestimmten Ausgangssignal eines Aktivitäts- oder Blutsauerstoffsättigungssensors entspricht, oder bei welchen Grenz-Herzraten eine vorgegebene Stimulation zur Behandlung einer Bradykardie oder Tachykardie einsetzt bzw. von einem Stimulationsmodus in einen anderen umgeschaltet wird. Diese zu einem bestimmten Zeitpunkt und aufgrund eines spezifischen körperlichen Zustands des Patienten getroffene Zuordnung muß jedoch keineswegs dauerhaft gültig sein. Insbesondere können Einflüsse, die sich in der Meßgröße nicht widerspiegeln (etwa Erkrankungen anderer Organe, psychische Faktoren. Änderungen der Lebensbedingungen), dazu führen, daß die vom Gerät gelieferte Therapie nach einiger Zeit nicht mehr optimal ist.

Durch die im wesentlichen gleichzeitige Erfassung und Auswertung der Signale mehrerer Sensoren kann zwar der aktuelle körperliche Zustand des Patienten zunehmend genauer abgebildet werden, dies ändert jedoch nichts an der nachteiligen strengen Determiniertheit der Therapie durch die Meßgröße(n).

Aus EP 0 841 074 und WO 96 02185 sind Therapiegeräte bekannt, in denen den ausgegebenen Therapiegrößen Schwankungen aufprägbar sind.

Der Erfindung liegt die Aufgabe zugrunde, ein Therapiegerät der eingangs genannten Gattung so weiterzubilden, daß dieses aufgrund eines vorgegebenen Auswertungs- und Steueralgorithmus eine gegenüber bekannten Geräten an sich ändernde körperliche Bedürfnisse des Patienten besser angepaßte Therapie zu liefern vermag, ohne daß das Gerät wesentlich komplexer und kostenaufwendiger wird.

Diese Aufgabe wird durch ein Therapiegerät mit den Merkmalen des Anspruchs 1 gelöst.

Die Erfindung schließt den Gedanken ein, ein Gerät mit Mitteln zu schaffen, die die strenge Determiniertheit der Therapie bzw. Therapiegröße durch die Meßgröße(n) dadurch relativieren, daß letzterer (letzteren) im Zuge der Auswertung eine willkürliche zeitliche Schwankung aufgeprägt wird - wodurch sich eine mit dem Schwankungswert korrigierte Pseudo-Meßgröße ergibt, deren weitere Auswertung mit dem festgelegten Algorithmus zur Ausgabe einer variierten Therapie (Therapiegröße) führt. Diese Variation der Therapie ermöglicht eine vergleichende Erfolgskontrolle, auf deren Grundlage (ohne Änderung des Algorithmus) die Therapie optimiert werden kann.

Die Erfolgskontrolle kann insbesondere mittels eines zusätzlichen, den körperlichen Zustand des Patienten insoweit, wie das Therapiegerät zu dessen Beeinflussung eingesetzt wird, möglichst genau abbildenden Sensors erfolgen. Alternativ hierzu kann sie über den auch zur Gewinnung der primären Meßgröße genutzten Sensor ausgeführt werden jeweils unter Zuhilfenahme von vorab in Zuordnung zueinander gespeicherten Meßwerten und Bewertungskriterien. Die erfolgreichere bzw. erfolgreichste der variierten Therapie(n) wird dann als aktuell gültige festgelegt. Dabei können - je nach der konkreten Aufgabe und Ausbildung des Therapiegerätes - verschiedene Strategien verfolgt und entsprechende technische Mittel eingesetzt werden, worauf weiter unten genauer eingegangen wird.

Für einige Ausführungsformen sind spezielle Mittel zur vergleichenden Erfolgskontrolle sogar verzichtbar, sei es, weil die Wirkung einer einmalig variierten Therapie eine (mindestens zeitweilige) selbsttätige Aussetzung jedweder Therapie zur Folge hat - man denke an eine aufgrund des schwankungsbehafteten Meßwertes der Herzrate ausgegebene, wirksame Impulsfolge zur Beendigung einer sich gerade beschleunigenden Tachykardie oder einen auf analoge Weise gesteuerten Kardioversionsimpuls bei einsetzenden Fibrillationen - oder weil eine fortgesetzte leichte Schwankung der Therapiegröße aufgrund der der Meßgröße aufgeprägten Schwankung aus grundsätzlichen Erwägungen als vorteilhaft angesehen werden kann, wie unter bestimmten Umständen bei einem Medikamentendosiergerät.

Bei einer zweckmäßigen Ausbildung mit einem einzigen Sensor zur Steuerung und zugleich zur Erfolgskontrolle ist dem Sensor ein Vergleichswertspeicher zugeordnet und eine eingangsseitig mit den Ausgängen des Sensors und des Vergleichswertspeichers verbundene Vergleichereinheit vorgesehen, in der der Sensor-Meßwert einem Vergleich mit mindestens einem gespeicherten Vergleichswert unterzogen wird und die im Ergebnis des Vergleiches ein Steuersignal abgibt, aufgrund dessen der Sensor-Meßwert entweder dem Eingang der mathematischen Verarbeitungseinheit - zur Verarbeitung mit einem Schwankungswert - oder unter Umgehung dieser direkt dem Eingang der Auswertungs- und Steuereinrichtung zugeführt wird.

Bei einem Zwei-Sensoren-Gerät hingegen sind ein erster und ein zweiter Sensor vorgesehen, wobei dem Ausgang des ersten Sensors der Schwankungswertgenerator zugeordnet ist derart, daß mit dem Schwankungswert korrigierte Meßwerte des ersten Sensors der Variation der Therapie bzw. Therapiegröße zugrundegelegt werden, während der Ausgang des zweiten Sensors mindestens mittelbar mit einem Eingang der Zeitsteuereinheit verbunden ist derart, daß die Aufprägung der Schwankungsgröße auf die Meßwerte des ersten Sensors in Abhängigkeit von den Meßwerten des zweiten Sensors gesteuert, insbesondere wahlweise unterbunden, wird.

Der oben genannte Sensor bzw. erste Sensor kann zur Erfassung einer Aktivitätsgröße oder einer eine Organfunktion des Patienten kennzeichnenden Größe und/oder zur Erfassung der Therapiegröße - etwa als intrakardiale Elektrode mit nachgeschaltetem Abfühlverstärker für elektrische Aktivität des Herzens, speziell mit zugeordneter Einrichtung zur Bestimmung der Periode der elektrischen Herzaktivität als Meßwert - ausgebildet sein. Der zweite Sensor wird bevorzugt ein zur Erfassung einer von einer Organfunktion oder der Therapiegröße abhängigen, eher den körperlichen bzw. hämodynamischen Gesamtzustand des Patienten kennzeichnenden Größe ausgebildeter Fühler, beispielsweise ein Blutdruckfühler, sein.

Die Schwankungswert-Verarbeitung der Meßgröße kann - je nach konkreter Anwendung - in zweckmäßiger Weise durch Addition/Subtraktion oder durch Multiplikation/Division erfolgen. Entsprechend ist der Schwankungswertgenerator zur Ausgabe mindestens eines Inkrement- oder Dekrementwertes und die mathematische Verarbeitungseinheit als Additionsstufe oder der Schwankungswertgenerator zur Ausgabe mindestens eines Korrekturfaktors und die mathematische Verarbeitungseinheit als Multiplikationsstufe ausgebildet. Spezieller umfaßt der Schwankungswertgenerator einen Schwankungswertspeicher für mehrere Schwankungswerte und wahlweise einen Zufallsgenerator zur Auswahl je eines der gespeicherten Schwankungswerte zur Aufprägung auf den Meßwert.

In der Ausbildung des Gerätes als frequenzadaptiver implantierbarer Herzschrittmacher ist die Auswertungs- und Steuereinrichtung zur Festlegung der Rate elektrischer Stimulationsimpulse und die Therapieeinrichtung zur Erzeugung und Abgabe der elektrischen Stimulationsimpulse mit der festgelegten Rate als Therapiegröße an das Herz ausgebildet, wobei der bzw. der erste Sensor zur Erfassung einer die körperliche Aktivität des Patienten repräsentierenden Größe als Meßwert ausgeführt sein kann.

In der weiteren Ausbildung als, insbesondere implantierbarer, Kardioverter kann zweckmäßigerweise der bzw. der erste Sensor durch eine intrakardiale Elektrode mit nachgeschaltetem Abfühlverstärker gebildet sein und eine Einrichtung zur Bestimmung der Periode der elektrischen Herzaktivität als Meßwert aufweisen. Die Auswertungs- und Steuereinrichtung ist hierbei zur Festlegung mindestens einer vorgegebenen Folge elektrischer Stimulationsimpulse und/oder eines energiereichen Einzelimpulses und die Therapieeinrichtung zur Erzeugung und Abgabe der entsprechenden elektrischen Stimulationsimpulse ausgebildet.

Weiterhin ist eine - die wesentlichen Merkmale beider Geräte vereinende - Ausführung als implantierbarer kombinierter Herzschrittmacher/Kardioverter möglich.

Eine ganz andersartige Realisierung stellt die Ausbildung als, insbesondere implantierbares, Medikamentendosiergerät dar, bei dem der bzw. der erste Sensor zur Erfassung des Pegels eines Wirkstoffes oder einer davon abhängigen Größe im Körper des Patienten, die Auswertungs- und Steuereinrichtung zur Festlegung einer Medikamentendosis pro Zeiteinheit und die Therapieeinrichtung zur Abgabe der festgelegten Dosis pro Zeiteinheit an den Körper ausgebildet sind. In einer speziellen Ausführung zur medikamentösen Behandlung kardialer Arrythmien kann die Meßwerterfassung speziell auch hier über eine intrakardiale Elektrode erfolgen, da die Herzaktionen natürlich die zu behandelnde Arrythmie ebenso reflektieren wie den Behandlungserfolg.

Vorteilhafte Weiterbildungen der Erfindung sind in den Unteransprüchen gekennzeichnet bzw. werden nachstehend zusammen mit der Beschreibung bevorzugter Ausführungen der Erfindung anhand der Figuren näher dargestellt. Es zeigen:
- Figur 1: ein stark vereinfachtes Funktions-Blockschaltbild eines Herzschrittmachers mit einem Sensor gemäß einer Ausführungsform der Erfindung,
- Figur 2: ein stark vereinfachtes Funktions-Blockschaltbild eines Herzschrittmachers mit zwei Sensoren gemäß einer weiteren Ausführungsform der Erfindung,
- Figur 3: ein stark vereinfachtes Funktions-Blockschaltbild eines Medikamentendosiergerätes für Antiarrhythmika mit einem Sensor gemäß einer weiteren Ausführungsform der Erfindung,
- Figur 4: eine schematische Darstellung des in verschiedene Abschnitte gegliederten Herzratenkontinuums zur Illustration der Arbeitsweise eines kombinierten Herzschrittmachers/Defibrillators nach einer weiteren Ausführungsform der Erfindung,
- Figur 5: ein stark vereinfachtes Blockschaltbild eines kombinierten Herzschrittmachers/Defibillators gemäß einer auf Fig. 4 bezogenen Ausführungsform der Erfindung und
- Figur 6: eine schematische Darstellung zum Betrieb des kombinierten Herzschrittmachers/Defibrillators nach Fig. 5.

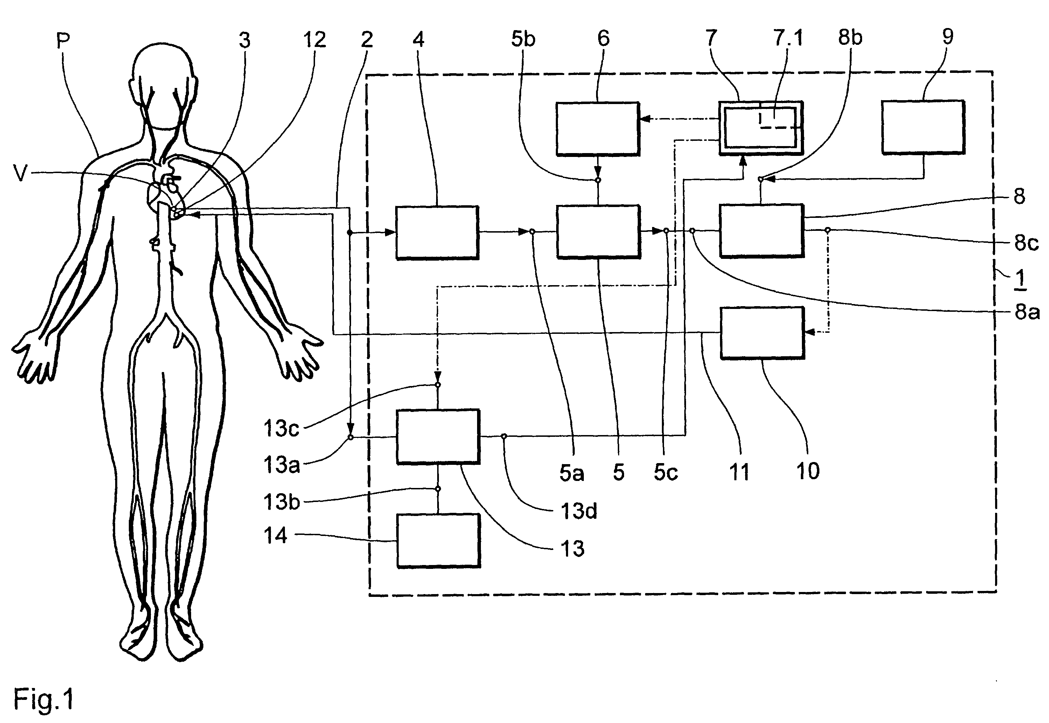

Anhand von Fig. 1 soll das Prinzip der Erfindung zunächst anhand einer - stark vereinfachten - Abwandlung eines aktivitätsgesteuerten Herzschrittmachers illustriert werden. Fig. 1 ist ein vereinfachtes Funktions-Blockschaltbild der für die Erläuterung der Erfindung wesentlichen Funktionselemente eines ratenadaptiven implantierbaren Herzschrittmachers 1, der über eine Ansteuer- und Meßleitung 2 mit einem im Ventrikel V des Herzens eines Patienten P angeordneten Sensor 3 für die Blutsauerstoffsättigung verbunden ist. Herkömmliche, dem Fachmann geläufige Bauteile eines Schrittmachers, die nicht in speziellem Bezug zur Ausführung der Erfindung stehen, wie die Stromversorgung und die Ausgangsstufe, sind in der Figur weggelassen und werden im weiteren nicht erläutert.

Der Sensor 3 und dessen Ansteuerung können gemäß dem seit längerem bekannten Prinzip des intrakadialen Pulsoximeters aufgebaut sein. Das proximale Ende der Meßleitung 2 ist mit dem Eingang einer Signalaufbereitungsstufe 4 verbunden, die (in an sich ebenfalls bekanntem Aufbau) Filterund Verstärkerstufen aufweist und an deren Ausgang ein weitgehend störbefreites Signal anliegt, das die aktuelle intrakardiale Blutsauerstoffsättigung des Patienten P repräsentiert. Da diese bekanntlich bei einer Erhöhung der körperlichen Aktivität (unter der Voraussetzung konstanter Herzleistung) absinkt, bei einer Verringerung der Aktivität hingegen zunimmt, reflektiert sie (mit einer gewissen Verzögerung und zeitlichen Mittelung) das Niveau der körperlichen Aktivität und kann daher zur Ratensteuerung des Schrittmachers 1 herangezogen werden.

Der Ausgang der Signalaufbereitungsstufe 4 ist mit einem ersten Eingang 5a einer Multiplikationsstufe 5 verbunden, die einen zweiten Eingang 5b aufweist, welcher mit dem Ausgang eines Korrekturfaktorspeichers 6 verbunden ist. Der Korrekturfaktorspeicher 6 ist weiterhin über einen Steuerund Adreßeingang mit dem Ausgang einer Programmsteuereinheit 7 verbunden, welche eine Zeitgeber 7.1 aufweist. Unter Steuerung durch diese werden aus dem Korrekturfaktorspeicher 6 sequentiell nach einem vorgewählten Zeitregime gespeicherte Korrekturwerte für das Blutsauerstoff-Meßsignal über deren zweiten Eingang 5b in die Multiplikationsstufe 5 gelesen und dort mit dem über deren ersten Eingang 5a zugeführten aktuellen Meßwert multipliziert. Das Produkt wird am Ausgang 5c ausgegeben.

Dieser Ausgang der Multiplikationsstufe 5 ist mit einem ersten Eingang 8a einer Auswertungs- und Therapiesteuereinheit 8 verbunden, die weiterhin einen zweiten, mit einem Therapie-Zuordnungsspeicher 9 verbundenen Eingang 8b aufweist. Der Therapie-Zuordnungsspeicher 9 kann als Programmspeicher zur Speicherung eines Algorithmus zur Berechnung einer Therapiegröße - hier speziell der Stimulationsrate des Schrittmachers - aus einer Meßgröße - hier der Blutsauerstoffsättigung - organisiert sein. Bevorzugt ist er aber als Direktzugriffs-Datenspeicher (RAM) aufgebaut, in dem in Tabellenform eine Werte-Zuordnung MeßwertTherapiegrößenwert (mithin hier: Wert der Blutsauerstoffsättigung - Wert der Stimulationsrate) gespeichert ist und der mit dem am ersten Eingang 8a der Auswertungs- und Therapiesteuereinheit 8 anliegenden Meßwert bzw. korrigierten Meßwert (hier: dessen Produkt mit dem Korrekturfaktor) adressiert wird. Der jeweils aus dem adressierten Speicherplatz ausgelesene Stimulationsratenwert wird der Ermittlung einer zugehörigen geräteinternen Steuergröße zugrundegelegt, die am Ausgang 8c der Einheit 8 bereitgestellt wird.

Der Ausgang 8c ist mit einem Steuereingang eines - als solchen wiederum bekannten - Schrittmacherimpulsgenerators 10 verbunden, der an seinem Ausgang Stimulationsimpulse mit der im Ergebnis der obigen Auswertung bestimmten Rate und programmierten übrigen Parametern (Impulsamplitude und - breite etc.) bereitstellt. Diese werden über eine Elektrodenleitung 11 und eine im Ventrikel V angeordnete Stimulationselektrode 12 dem Herzen zugeführt.

Die entsprechend der Stimulationsrate ablaufende - je nach Richtung der initialen Änderung der Blutsauerstoffsättigung beschleunigte oder verlangsamte - Herztätigkeit wirkt (vereinfacht dargestellt) der Änderung des Sauerstoffgehaltes im Sinne der Zurückführung auf einen Normal- oder Gleichgewichtswert entgegen. Dieser Prozeß kann beim vorliegenden Beispiel zugleich mittels des Sensors 3 verfolgt werden, der die Meßwerte für die Steuerung liefert.

Hierzu ist eine über einen ersten Dateneingang 13a mit dem Sensor 3 verbundene Kontroll-Auswertungseinheit 13 vorgesehen, die über einen zweiten Dateneingang 13b mit einem Sollwertspeicher 14 verbunden ist, in dem ein patientenspezifischer Normalwert der intrakardialen Blutsauerstoffsättigung gespeichert ist. Dieser dient als Bezugswert für eine Erfolgskontrolle der Schrittmachertherapie mit der Stimulationsrate als variierter Therapiegröße. Über einen Steuereingang 13c ist die Kontroll-Auswertungseinheit 13 mit der Programmsteuereinheit 7 verbunden, wodurch Synchronität der Kontrollauswertung mit dem Ablauf der Schwankungswert-Verarbeitung der Meßgröße gewährleistet und gleichzeitig die für die Kontrollauswertung benötigte Zeitbasis bereitgestellt wird. Die Kontrollauswertung kann (wiederum sehr vereinfacht dargestellt) einen Vergleich der zeitlichen Ableitung der Differenz zwischen aktueller Sauerstoffsättigung und Sollwert (a) in den Zeitphasen, in denen zur Festlegung der Stimulationsrate vom unkorrigierten Meßwert ausgegangen wird, mit (b) der Zeitableitung in den Zeitphasen, in denen mit dem schwankungsbehafteten Meßwert gearbeitet wurde, umfassen. Ergibt sich bei der Kontrollauswertung, daß die Angleichung der Blutsauerstoffwerte an den Normalwert tendenziell schneller erfolgt, wenn der tatsächliche Meßwert mit dem Schwankungswert (Korrekturfaktor) beaufschlagt wird, wird über den Ausgang 13d der Kontroll-Auswertungseinheit ein entsprechendes Signal an die Programmsteuereinheit 7 ausgegeben, woraufhin diese den als vorteilhaft ermittelten Korrekturfaktor permanent bereitstellt. Die weitere Steuerung des Schrittmachers in dieser Betriebsperiode erfolgt mithin dann mit einem korrigierten statt mit dem tatsächlichen Meßwert. Entsprechend wird, wenn die Steuerung mit dem tatsächlichen Meßwert sich als günstiger erwiesen hat, die Zuschaltung des Korrekturfaktors durch die Programmsteuereinheit 7 im weiteren unterbunden.

Die oben erläuterte Betriebsweise kann bei jedem durch eine Änderung der gemessenen Blutsauerstoffsättigung initiierten Steuervorgang ablaufen. Die Programmierung des Schrittmachers kann aber auch derart sein, daß sie nur in bestimmten Zeitabständen - d.h. in Testphasen - aktiviert wird, während in der übrigen Zeit die Meßwerte ohne Beaufschlagung mit dem Korrekturfaktor auf die übliche Weise verarbeitet werden. Das letztere Vorgehen ist im Kontext der Erfindung insbesondere dann vorteilhaft, wenn ein in einer Testphase mit positivem Ergebnis verwendeter willkürlicher Korrekturfaktor bis zur nächsten Testphase dem tatsächlichen Meßwert permanent zugeordnet wird. Die nächste Testphase kann dann wieder - im Sinne einer Verifizierung - mit demselben Korrekturfaktor ausgeführt werden, es kann dann aber auch (neben dem "Rückwärts-Abgleich" mit dem unkorrigierten Meßwert) ein "Vorwärts-Abgleich" mit einem - oder auch mehreren - weiteren Korrekturfaktor(en) eingeschlossen sein. Dies setzt natürlich eine entsprechende Ausbildung bzw. Arbeitsweise des Korrekturfaktorspeichers 6, der Programmsteuerreinheit 7 und der Kontroll-Auswertungseinheit 13 voraus und findet überdies eine praktische Grenze in der Trägheit des metabolischen Systems. Grundsätzlich jedoch wird in der Abfolge solcher Testphasen der Schrittmacher 1 dann als selbstlernendes Gerät betrieben.

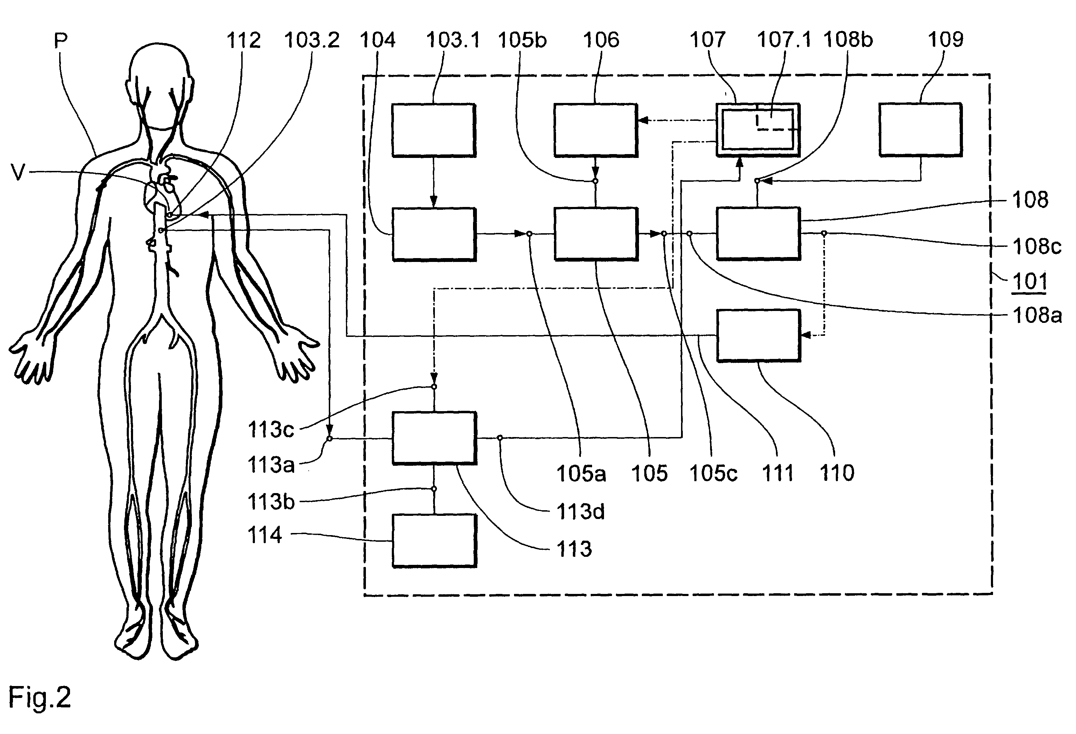

Figur 2 zeigt in einem an Fig. 1 angelehnten, ebenfalls stark vereinfachten Funktions-Blockschaltbild einen ratenadaptiven Herzschrittmacher 101 mit Zwei-SensorenSteuerung gemäß einer weiteren Ausführungsform der Erfindung. Soweit dieser mit dem Schrittmacher nach Fig. 1 funktionsgleiche Baugruppen aufweist, sind diese mit ähnlichen Bezugsziffern bezeichnet und werden im weiteren nicht nochmals erläutert.

Der Schrittmacher 101 weist einen in das Schrittmachergehäuse eingebauten piezoelektrischen Aktivitätssensor 103.1 auf und ist weiterhin über eine Meßleitung 102 mit einem intravaskulären Blutdrucksensor 103.2 verbunden. Beide Sensoren sind als solche bekannt. Der Ausgang des Aktivitätssensors 103.1 ist mit dem Eingang einer Signalaufbereitungsstufe 104 verbunden, die (in an sich ebenfalls bekanntem Aufbau) Filter- und Verstärkerstufen aufweist und an deren Ausgang ein (pegelmäßig an die Arbeitsspannungen der nachfolgenden Verarbeitungsstufen angepaßtes) Signal anliegt, das das Niveau der aktuellen körperlichen Aktivität des Patienten P repräsentiert.

Die Verarbeitung der Aktivitätssignale kann analog zu Fig. 1 erfolgen; nachfolgend wird jedoch eine modifizierte Verarbeitung erläutert: An die Stelle der Multiplikationsstufe 5 in Fig. 1 tritt dabei eine Additionsstufe 105 mit Meßsignaleingang 105a, und an die des Korrekturfaktorspeichers 6 ein Inkrementspeicher 106. Unter Steuerung durch eine Programmsteuereinheit 107, welche einen Zeitgeber 107.1 aufweist, werden aus dem Inkrementspeicher 106 sequentiell ein oder - nach einem vorgewählten Zeitregime mehrere gespeicherte additive (r) Korrekturwert(e) für das Aktivitäts-Meßsignal über deren zweiten Eingang 105b in die Additionsstufe 105 gelesen und dort zu dem über deren ersten Eingang 105a zugeführten aktuellen Meßwert addiert. Die Summe (bzw. zwischenzeitlich auch jeweils der unkorrigierte Meßwert) wird am Ausgang 105c ausgegeben.

Der weitere Aufbau und die Verarbeitung des phasenweise korrekturwertbehafteten Meßsignals sind analog zur Anordnung nach Fig. 1.

Die entsprechend der Stimulationsrate ablaufende - je nach dem zeitlichen Verlauf der Aktivitätsgröße beschleunigte oder verlangsamte - Herztätigkeit und die entsprechend gesteuerte Herzleistung (cardiac output) wirkt sich auf den mittleren arteriellen Blutdruck aus, der in gewissem Sinne als Regelgröße des hämodynamischen Regelsystems des Menschen aufgefaßt werden kann. Die Messung des mittleren arteriellen Blutdrucks in Abhängigkeit von der Zeit eröffnet somit eine Möglichkeit der Erfolgskontrolle über die aktivitätsgesteuerte Stimulationsratenanpassung: Je besser der Blutdruck konstant gehalten wird (d.h. auch, je schneller Schwankungen aufgrund veränderter Aktivität ausbalanciert werden), desto effizienter ist die Frequenzanpassung der Stimulationsrate.

Zur Ausführung dieser Erfolgskontrolle - die im übrigen grundsätzlich so abläuft, wie oben unter Bezugnahme auf Fig. 1 beschrieben - ist bei der Anordnung nach Fig. 2 eine über einen ersten Dateneingang 113a mit dem Blutdruckfühler 103.2 verbundene Kontroll-Auswertungseinheit 113 vorgesehen, die über einen zweiten Dateneingang 113b mit einem Sollwertspeicher 114 verbunden ist, in dem ein patientenspezifischer Blutdruck-Normalwert als Bezugswert gespeichert ist. Ergibt sich bei der Kontrollauswertung, daß der Blutdruck tendentiell stabiler ist, wenn das Aktivitäts-Meßsignal für die weitere Verarbeitung mit dem (bzw. einem der verfügbaren) Inkrementwert(e) korrigiert wird, wird über den Ausgang 113d der Kontroll-Auswertungseinheit 113 ein entsprechendes Signal an die Programmsteuereinheit 107 ausgegeben, woraufhin diese den als vorteilhaft ermittelten Inkrementwert permanent bereitstellt. Die weitere Steuerung des Schrittmachers in dieser Betriebsperiode erfolgt mithin dann mit einem korrigierten statt mit dem tatsächlichen Aktivitätssignal. Andererseits wird, wenn die Stimulierung in den Perioden, in denen mit dem ursprünglichen Aktivitätssignal gearbeitet wurde, sich als effizienter erwiesen hat, die Zuschaltung des Korrektur-Inkrementes im weiteren unterbunden.

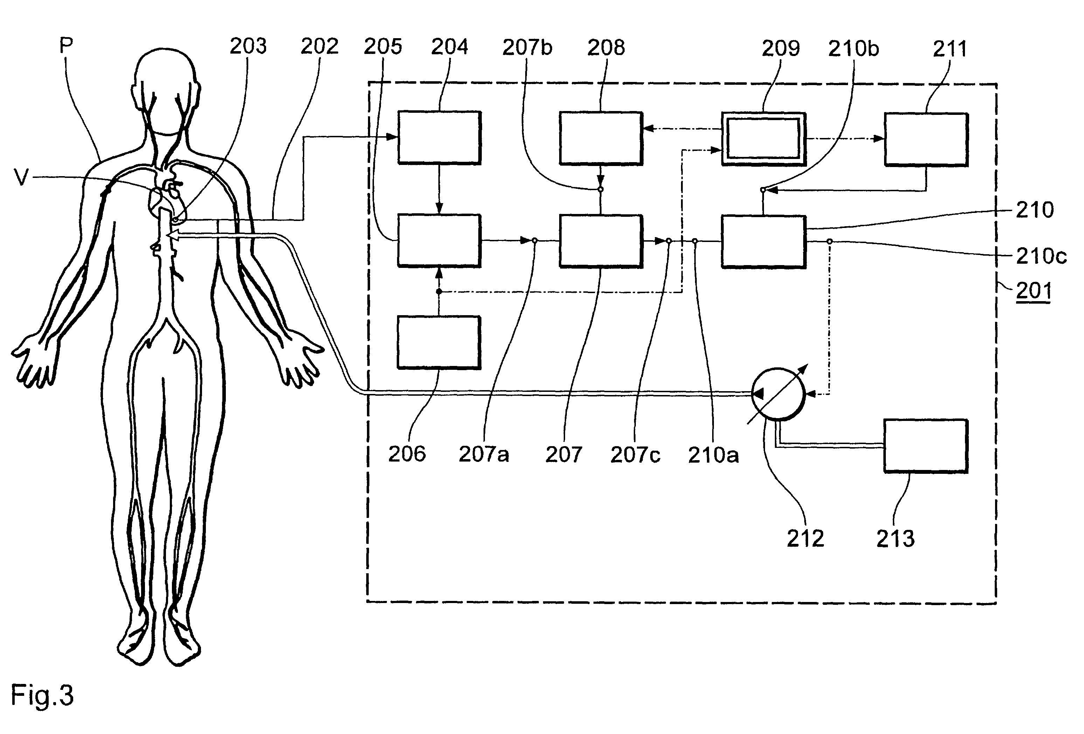

Figur 3 ist ein - wie schon die vorigen Figuren - stark vereinfachtes Funktions-Blockschaltbild eines Medikamentendosiergerätes 201, speziell für Medikamente zur Korrektur von Herzarrhythmien. Die nachfolgend beschriebene Ausführung eignet sich grundsätzlich zur Steuerung einer selbsttätigen medikamentösen Behandlung von plötzlich auftretenden lebensbedrohlichen Bradykardien oder Tachykardien mittels geeigneter Antiarrhythmika.

Das Gerät 201 ist über eine Elektrodenleitung 202 mit einer im Ventrikel V eines Herzens H positionierten intrakardialen Herzelektrode 203 zur Aufnahme von Herzaktionspotentialen verbunden. Das proximale Ende der Elektrodenleitung 202 ist mit einer Eingangsverstärkerstufe 204 verbunden, die (in an sich von Herzschrittmachern bekanntem Aufbau) Filter- und Verstärkerstufen zur Signalaufbereitung aufweist und an deren Ausgang ein weitgehend störbefreites, pegelangepaßtes Herzsignal anliegt. Der Ausgang der Eingangsstufe 204 ist mit dem Signaleingang einer Zählerstufe 205 verbunden, deren Takteingang mit einem Taktbzw. Zeitgeber 206 verbunden ist und in der die Rate fRR der erfaßten Herzaktionen (Ventrikeldepolarisationen bzw. R-Wellen) ermittelt wird.

Ähnlich wie bei der Anordnung nach Fig. 2, ist der Ausgang der Zählerstufe 205 über einen Meßsignaleingang 207a mit einer Additionsstufe 207 verbunden, und es ist ein Inkrement-/Dekrementspeicher 208 für additiv oder subtraktiv zu berücksichtigende (d.h. positive oder negative) Herzraten-Korrekturwerte vorgesehen. Unter Steuerung durch eine Programmsteuereinheit 209, die mit dem Taktgeber 206 verbunden ist, werden aus dem Datenspeicher 208 sequentiell nach einem vorgewählten Zeitregime mehrere gespeicherte Korrekturwerte für den aktuellen Wert der Herzrate fRR über deren zweiten Eingang 207b in die Additionsstufe 207 gelesen und dort zur Korrektur des über deren ersten Eingang 207a zugeführten aktuellen Meßwerts eingesetzt. Die Summe oder Differenz (bzw. zwischenzeitlich auch jeweils der unkorrigierte Meßwert) wird am Ausgang 207c ausgegeben.

Der Ausgang 207c ist mit einem ersten Signaleingang 210a einer Auswertungs- und Pumpenansteuereinheit 210 verbunden, die über einen zweiten Signaleingang 210b mit einem Dosierdatenspeicher 211 in Verbindung steht. Aufbau und Organisation dieses Speichers entsprechen im wesentlichen denen des Speichers 9 aus Fig. 1, wobei im vorliegenden Beispiel eine Zuordnungstabelle Herzrate - Medikamentendosis gespeichert ist. Der jeweils aus dem adressierten Speicherplatz ausgelesene Dosiswert wird der Ermittlung einer zugehörigen geräteinternen Steuergröße zugrundegelegt, die am Ausgang 210c der Auswertungs- und Pumpenansteuereinheit 210 bereitgestellt wird. Dieser ist mit einer Dosierpumpe 212 verbunden, über die ein in einem Medikamententank 213 gespeichertes Antiarrhythmikum mit der berechneten Dosierung in den Körper des Patienten P abgegeben werden kann. Bei einem gefährlichen Abfallen oder Ansteigen der Herzrate wird somit automatisch eine medikamentöse Behandlung eingeleitet, deren Erfolg anhand der über die Herzelektrode 203 aufgenommenen Signale kontrollierbar ist.

Die sich entsprechend der Wirkung des injizierten Medikamentes ergebende, über die Elektrode 203, die Eingangsstufe 204 und die Zählerstufe 205 erfaßte, aktuelle Herzrate wird wieder der oben beschriebenen Verarbeitung unterzogen. Beim vorliegenden Beispiel ist zu beachten, daß das Dosiergerät 201 eine Notfalltherapie bewirken soll, d.h. die Dosierpumpe 212 im Normalfall außer Betrieb sein soll. Entsprechend sind auch die vorgespeicherten Inkrementbzw. Dekrementwerte und die Zuordnungstabelle für Herzrate und Medikamentendosis in Abstimmung aufeinander derart zu wählen, daß eine Medikamentenabgabe nur bei gemessenen wie auch korrekturbehafteten Werten der Herzrate erfolgt, die einem für den Patienten mit hoher Wahrscheinlichkeit kritischen Bereich zuzuordnen sind.

Eine Erfassung und Speicherung eines therapeutisch vorteilhaften Inkrement- oder Dekrementwertes ist hier verzichtbar, da es bei der Notfalltherapie weniger darauf ankommt, ob der ursprüngliche oder einer (und welcher) der variierten Herzraten-Meßwerte der Ausgangspunkt für eine wirksame medikamentöse Behandlung war, als vielmehr entscheidend darauf, daß diese effizient war. Zudem können sich bis zu einem eventuellen erneuten Bradykardie- oder Tachykardienanfall die patientenseitigen Bedingungen erheblich ändern, so daß die Variation der Therapiesteuerung dann ggfs. zweckmäßigerweise vom gleichen "neutralen" Ausgangspunkt - nämlich dem aktuellen Meßwert - aus erfolgen sollte.

Nachfolgend soll die Erfindung weiter am Beispiel einer Anordnung zur differenzierten Elektrostimulations-Therapie verschiedener Formen von Herzarrhythmien, eines kombinierten Herzschrittmachers/Defibrillators, erläutert werden.

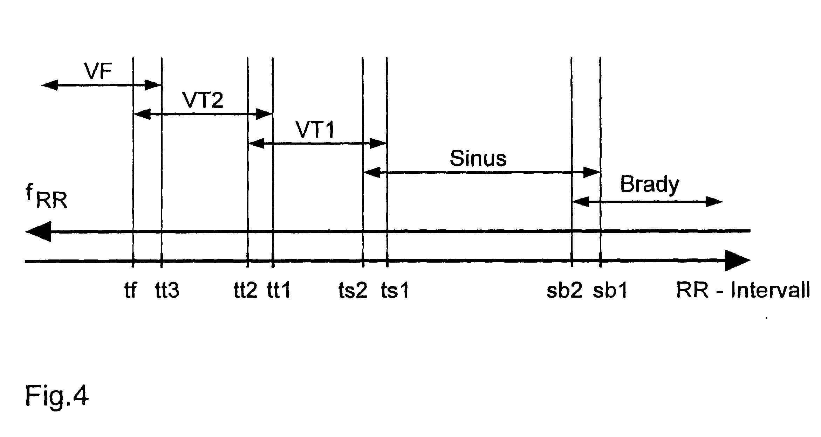

Figur 4 ist zunächst eine schematische Darstellung des in verschiedene, einander überlappende Abschnitte gegliederten Herzratenkontinuums zur Illustration der Arbeitsweise eines solchen Gerätes. Auf der x-Achse ist (mit nach rechts zunehmenden Werten) des RR-Intervall bzw. (mit nach links zunehmenden Werten) die Herzrate fRR aufgetragen. Mit "VF" ist der Bereich ventrikulärere Fibrillation bezeichnet, mit "VT2" und "VT!" sind zwei aneinander angrenzende Bereiche ventrikulärer Tachykardie mit unterschiedlicher diagnostischer und therapeutischer Relevanz bezeichnet, "Sinus" bezeichnet den Bereich normaler Herztätigkeit und "Brady" den Bereich unzulässig niedriger Herzrate, d.h. einer (ventrikulären) Bradykardie. Grundsätzlich erfordert die Messung eines Wertes von fRR außerhalb des Bereiches "Sinus" eine spezifische Stimulation des Herzens zur Zurückführung in den Normalbereich, wobei die Art und Weise der Stimulation und deren physiologische Auswirkungen auf den Patienten sich erheblich unterscheiden.

Mit "tf", "tt3", "tt2", "tt1", "ts2", "ts1", "sb2" und "sb1" sind die (wegen der erwähnten Überlappung jeweils doppelten) Grenzen zwischen den einzelnen Bereichen bezeichnet; zur Bedeutung der Überlappungsregionen siehe weiter unten.

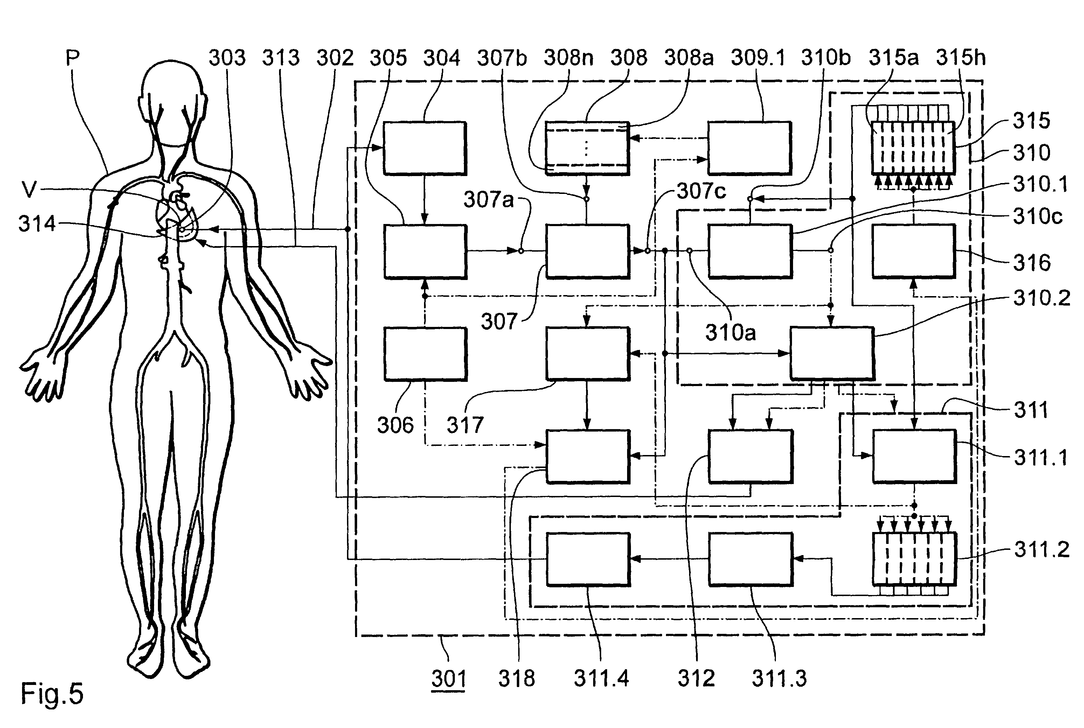

Figur 5 ist ein stark vereinfachtes Blockschaltbild eines kombinierten Herzschrittmachers/Defibillators 301 gemäß einer auf Fig. 4 bezogenen Ausführungsform, der - in an sich bekannter Weise - über eine Elektrodenleitung 302 mit einer ventrikulär fixierten Herzelektrode 303 verbunden ist. Die über diese im Ventrikel V aufgenommenen Depolarisationssignale werden analog zur Anordnung nach Fig. 3 in den dortigen Blöcken 204 bis 209 entsprechenden Baugruppen 304 bis 309 weitergeleitet und verarbeitet. Die diesbezügliche Erläuterung zu Fig. 3 wird daher hier nicht wiederholt.

Es wird hier angenommen, daß der Speicher 308 über eine Anzahl von Speicherbereichen 308a bis 308n verfügt, in denen Inkrement-/Dekrementwerte innerhalb eines Streuungsbereiches von beispielsweise +/-25 ms gespeichert sind, und daß deren Adressierung auf statistische Weise über einen Zufallsgenerator 308.1 erfolgt. Der Streuungsbereich der Inkrement-/Dekrementwerte kann beispielsweise auch als Bruchteil des gemessenen RR-Intervalls definiert oder patientenspezifisch programmiert werden; wichtig ist jedoch, daß grundsätzlich die Steuerung der Therapie unter Bezugnahme auf einen konkret im bzw. am Körper erfaßten Meßwert erfolgt. Insbesondere gewährleistet der Einsatz eines Zufallsgenerators, wenn die adressierbaren Inkrement- und Dekrementwerte im Streuungsbereich gleichmäßig verteilt sind, daß der zeitliche Mittelwert des Korrekturbetrages Null ist.

Der Ausgang der Additionsstufe 307 ist über einen Signaleingang 310a einer Therapie-Vorwahleinheit 310 einerseits mit einem Steuereingang einer Schrittmacherstufe 311 und andererseits mit einem Steuereingang einer Kardioverterstufe 312 verbunden, so daß der am Ausgang der Stufe 307 anliegende RR-Intervall-Meßwert oder -Summenwert entweder zur Ansteuerung eines Schrittmachers (ähnlich wie oben unter Bezugnahme auf Fig. 1 beschrieben) zur Ausgabe anitbradykarder oder antitachykarder Impulsfolgen oder aber zur Auslösung eines hochenergetischen Schockimpulses (Defibrillations- oder Kardioversionsimpulses) dienen kann.

Der Ausgang der Schrittmacherstufe 311 ist über die Elektrodenleitung 302 mit der Ventrikelelektrode 303 verbunden, die somit eine Doppelfunktion als Abfühlelektrode des Kombinationsgerätes 301 und als Stimulationselektrode seiner Schrittmacherstufe 311 hat. Der Ausgang der Kardioverterstufe 312 ist über eine zweite, intrathorakal verlegte Elektrodenleitung 313 mit einer epikardial am Herzen H angeordneten Defibrillations-Flächenelektrode 314 verbunden, über die im Bedarfsfall die hohe Energie eines Defibrillationsschocks auf das reizbare Herzgewebe mit noch gewebsverträglicher Energiedichte übertragen wird.

Die Therapie-Vorwahleinheit 310 umfaßt einen Komparator 310.1, der über einen ersten Dateneingang mit der Additionsstufe 307 und über einen zweiten Dateneingang 310b mit zwei Speicherbereichen 315a, 315b eines Bereichsgrenzenspeichers 315 verbunden ist. In diesen sind die oben in Bezug zu Fig. 4 erwähnten beiden Grenzen tf und tt3 der Bereiche VF und VT2 im RR-Intervall- bzw. Herzratenkontinuum als patientenspezifisch vorprogrammierte Werte gespeichert. Dem Bereichsgrenzenspeicher 315 ist ein Adreßwähler 316 zugeordnet, der - wie weiter unten genauer erläutert wird - in Abhängigkeit von einem die TherapieVorgeschichte reflektierenden internen Steuersignal jeweils einen der beiden Speicherbereiche 315a, 315b mit dem Komparator 310.1 verbindet, den anderen aber sperrt. Der Komparator 310.1 ist ausgangsseitig mit einem Umschalter 310.2 verbunden, der in Abhängigkeit vom Ergebnis des Vergleiches des aktuellen Ausgangswertes der Additionsstufe 307 mit dem gespeicherten Bereichsgrenzwert tf oder tt3 die Schrittmacherstufe 311 oder die Kardioverterstufe 312 aktiviert und ggfs. zugleich den besagten Ausgangswert zur Schrittmacherstufe 311 durchschaltet.

Die Schrittmacherstufe 311 weist eine mehrstufige Vergleichereinheit 311.1 auf, die - in ähnlicher Weise wie die Therapie-Vorwahleinheit 310 - über zweite Eingänge mit Speicherbereichen 315c bis 316h des Bereichsgrenzenspeichers 315 verbunden ist, wobei auch hier wieder in Abhängigkeit von der Therapie-Vorgeschichte über den Adreßwähler 316 eine Adressierung oder aber Sperrung jeweils eines der Bereiche 315c (Wert tt2) oder 315d (Wert tt1), 315e (Wert ts2) oder 315f (Wert ts1) bzw. 315g (Wert sb2) oder 315h (Wert sbl) erfolgt. In der mehrstufigen Vergleichereinheit 311.1 wird der Ausgangswert der Additionsstufe 307 mit den verschiedenen Bereichsgrenzen verglichen und in Abhängigkeit vom Vergleichsergebnis am Ausgang der Vergleichereinheit 311.1 ein Signal ausgegeben, das die Zuordnung des Ausgangssignals der Stufe 307 zu einem der o.g. Herzraten- bzw. RR-Intervallbereiche ausdrückt.

Dieses Signal wird zur Adressierung einem ImpulsfolgenSteuersignalspeicher 311.2 zugeführt, woraufhin aus dem jeweils adressierten Speicherplatz ein vorgespeichertes Impulsfolgemuster (das sich insbesondere durch eine vorbestimmte Stimulationsrate, aber ggfs. auch durch einen bestimmten Aufbau und weitere Parameter auszeichnet) an einen Schrittmacher-Impulsgenerator 311.3 ausgegeben wird. Dieser erzeugt eine dem Impulsfolgemuster entsprechende Folge von Impulsen, die in üblicher Weise eine Ausgangsstufe 311.4 durchlaufen und über die Elektrode 303 an das Herz H abgegeben werden, falls die Schrittmacherstufe 311 aktiviert ist. Dies ist nach obigem der Fall, wenn das Ausgangssignal der Stufe 307 einem der Bereiche VT2, VT1 oder Brady zuzuordnen ist.

Ist hingegen im Falle eines im Ratenbereich VF liegenden Ausgangsignals der Stufe 307 die Kardioverterstufe 312 aktiviert, wird dort (in an sich bekannter Weise) ein einzelner Schockimpuls mit vorprogrammierten Parametern erzeugt und über die Elektrode 314 an das Herz H abgegeben.

Sowohl der Ausgang des Komparators 310.1 in der Therapie-Vorwahleinheit 310 als auch derjenige der mehrstufigen Vergleichereinheit 311.1 sind zusätzlich mit einem Meßwertspeicher 317 verbunden, der bei jeder - in den Vergleicherstufen 310.1 oder 311.1 ermittelten - Änderung der Zuordnung des Ausgangswertes der Additionsstufe 307 zu einem der oben erwähnten Bereiche des Herzraten- bzw. RRIntervall-Kontinuums zur Einspeicherung der jeweils aktuellen Zuordnung adressiert wird und in dem somit eine vorbestimmmte Anzahl von (in gewissem Sinne die TherapieVorgeschichte reflektierenden) Zuordnungen der Herzrate bzw. des RR-Intervalls aus der Vergangenheit gespeichert wird. Einfachstenfalls wird nur die Zuordnung vor der jeweils letzten Änderung gespeichert, in einer aufwendigeren, nach dem LI-FO(last-in-first-out)-Prinzip organissierten, Ausführung mit mehreren Speicherbereichen arbeitet der Speicher 317 als regelrechter Trendspeicher.

Der Meßwertspeicher 317 ist mit einem Eingang einer TrendAuswertungsstufe 318 verbunden, die über einen weiteren Eingang zudem mit dem Ausgang der Additionsstufe 307 verbunden ist und deren Ausgang mit dem Adreßwähler 316 in Verbindung steht. Die Trend-Auswertungsstufe 318 liefert im Ergebnis der Auswertung der zeitlichen Entwicklung der Herzraten- bzw. RR-Intervall-Zuordnung - ausgehend vom aktuellen Wert mindestens über eine Änderungsstufe hinweg das obe erwähnte Steuersignal für den Adreßwähler bzw. Pointer 316. Über diesen wird (gemäß obiger Erläuterung) wiederum die in den Überlappungsregionen tf-tt3, tt2-tt1, ts2-ts1 und sb2-sb1 aktuell gültige Bereichszuordnung des Ausgangssignals der Stufe 307 und damit weiter die gültige Therapie (Defibrillationsschock oder konkret anzuwendende Antitachykardie- oder Bedarfsstimulations-Impulsfolge) bestimmt.

Nachfolgend soll das Zusammenwirken der vorstehend beschriebenen Funktionseinheiten nach Fig. 5, insbesondere hinsichtlich der Therapiesteuerung in den Überlappungsregionen, anhand spezieller Situationen erläutert werden, die bei einem zu bestimmten tachykarden Rhythmusstörungen disponierten Patienten auftreten können, bei dem die Herzrate bzw. das RR-Intervall in die Bereiche VT1, VT2 oder VF fallen kann:

Grundsätzlich ist jedem der Bereiche über der RR-Achse in Fig. 4 eine spezifische Elektrostimulations-Therapie (d.h. ein Satz von Stimulationsparametern) zugeordnet - wobei zu beachten ist, daß unter "RR-Rate" bzw. "RR-Intervall" ein mit einem Schwankungs-Inkrement bzw. -Dekrement korrigierter Meßwert verstanden werden soll. Ändert sich der mit der Schwankungsgröße behaftete Meßwert (das Ausgangssignal der Additionsstufe 307) innerhalb eines Bereiches, wird die Therapie nicht geändert. Überschreitet er jedoch eine Bereichsgrenze, schaltet der in Fig. 5 schematisch dargestellte Schrittmacher/Kardioverter grundsätzlich auf eine andere einer Mehrzahl vorbestimmter Therapien um. Infolge der Behaftung mit der statistischen Schwankungsgröße erfolgt bei Meßwerten, die in den grenznahen Gebieten der Bereiche liegen, gelegentlich ein Umschalten zwischen verschiedenen Therapien, mit anderen Worten: die Schwankungsgröße ermöglicht - wie schon bei den weiter oben erläuterten Ausführungsformen nach Fig. 1 bis 3 - ein "Ausprobieren" verschiedener Therapien bzw. Therapiegrößen.

Kommt nun bei der vorliegenden Ausführung beispielsweise im Ergebnis einer Beschleunigung einer Tachykardie die (schwankungsbehaftete) Herzrate vom VT1-Bereich in die Überlappungsregion zum VT2-Bereich zwischen den Grenzwerten tt1 und tt2, wird auf die für den Bereich VT2 gültige ("aggressivere") Therapie umgeschaltet. Dies ist therapeutisch folgerichtig, da die im Bereich VT1 angewandte (weniger "aggressive") Therapie die Beschleunigung der Tachykardie nicht verhindern konnte, sich mithin als zu schwach erwies. Gelangt die Herzrate im Zuge der Verlangsamung einer ursprünglich im VT2-Bereich liegenden Tachykardie ebenfalls in die Überlappungsregion tt1-tt2, wird die für den VT2-Bereich gültige Therapie beibehalten. Auch dies ist folgerichtig, denn diese Therapie hat sich als erfolgreich erwiesen.

Springt hingegen die Herzrate aus dem Bereich Sinus in dieselbe Überlappungsregion tt2-tt1 der Bereiche VT1, VT2, so wird nicht die für den Bereich VT2, sondern die für den Bereich VT1 gültige Therapie (Impulsfolge) eingeschaltet. Dieses Vorgehen beruht auf der Überlegung, daß bei dieser Entwicklung der Körper des Patienten nicht übergangslos mit der "aggressiveren" Therapie für den Bereich VT2 belastet werden sollte, solange nicht versuchsweise die für den Bereich VT1 gültige Therapie angewandt worden ist. Damit soll auch das Risiko minimiert werden, durch die Therapie selbst eine weitere Beschleunigung der Tachykardie zu induzieren. Die für den Bereich VT2 gültige Therapie wird erst angewandt, wenn sich die Herzrate derart weiter erhöht, daß sie die Überlappungsregion verläßt.

Entsprechend differenziert wird die gültige Therapie auch bei einem entweder langsamen oder aber sprunghaften Übergang der Herzrate in die Überlappungsregion tf-tt3 zwischen den Bereichen VT2 und VF gewählt. Die Überlappungsregionen ts1-ts2 zwischen den Bereichen Sinus und VT1 und sb1-sb2 zwischen den Bereichen Brady und Sinus könen jedoch als "klassische" Hysteresebereiche behandelt werden, in denen mithin die Wahl der Therapie lediglich von der Richtung des Eintritts in die Überlappungsregion abhängt.

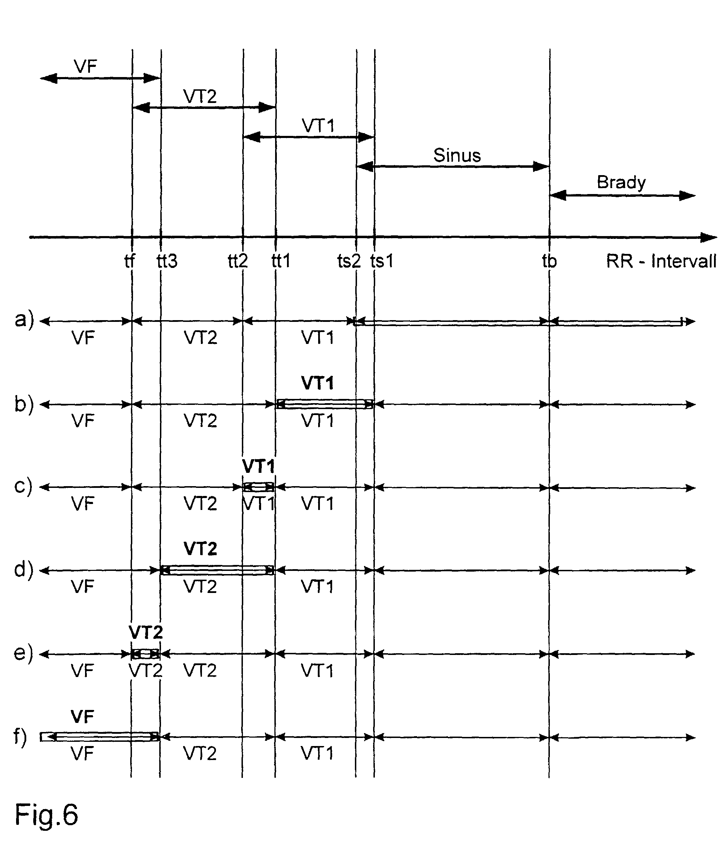

Die oben skizzierte Betriebsweise des Schrittmachers/Kardioverters gemäß der vorliegenden Ausführungsform ist in Fig. 6 illustriert, bei der die Bereichsunterteilung im wesentlichen Fig. 4 entspricht (lediglich der Überlappungsbereich sb1-sb2 zwischen den Bereichen Brady und Sinus ist hier weggelassen):
- Zeile a) zeigt, daß, wenn sich in einer ersten Phase die Herzrate in einem der Bereiche Brady oder Sinus befindet und keine Therapie bzw. eine Bradykardie-Stimulation angewandt wird, bei einer anschließenden Beschleunigung in den Raten-Bereich zwischen ts2 und tt1 die Therapie "VT1", zwischen tt2 und tf die Therapie "VT2" und oberhalb von tf die Therapie "VF" angewandt wird.
- Zeile b) zeigt, daß, wenn anfänglich die Rate im Gebiet zwischen ts1 und tt1 war und mit VT1 therapiert wurde (was für RR-Werte zwischen ts1 und ts2 nach obigem übrigens nur dann zutrifft, wenn diese Region im Zuge der Verlangsamung einer Tachykardie erreicht worden war), ein anschließender Übergang in das Gebiet zwischen tt1 und tf zur Anwendung der Therapie "VT2", ein Übergang auf einen Wert oberhalb tf aber zur Anwendung der Therapie "VF" führt. Eine Verlangsamung in den Bereich zwischen tb und ts1 hinein führt zum Aussetzen jeglicher Therapie und eine solche unter tb zu einer Bradykardie-Stimulation - was auch für die folgenden Zeilen gilt.
- Zeile c) zeigt, daß, wenn anfänglich die Rate im Gebiet zwischen tt1 und tt2 war und entsprechend die Therapie "VT1" angewandt worden war (was nach obigem nur dann der Fall sein wird, wenn die Region im Zuge einer sich schnell beschleunigenden Tachykardie erreicht wurde), ein anschließender Übergang in das Gebiet zwischen tt2 und tf zur Anwndung der Therapie "VT2", ein Übergang auf einen Wert oberhalb tf zur Anwendung der Therapie "VF", eine Verlangsamung der Tachykardie in das Gebiet zwischen ts1 und tt1 aber zur Beibehaltung der Therapie "VT1" führt.
- Zeile d) zeigt, daß, wenn anfänglich die Rate im Gebiet zwischen tt1 und tt3 war und die Therapie "VT2" angewandt worden war, ein anschließender Übergang auf einen Wert oberhalb tt3 zur Anwendung der Therapie "VF" führt. Eine Verlangsamung in den Bereich zwischen ts1 und tt1 hinein führt zur Umschaltung auf die Therapie "VT1". Letzteres gilt auch für die Zeilen e) und f).
- Zeile e) zeigt, daß, wenn anfänglich die Rate im Gebiet zwischen tt3 und tf war und entsprechend die Therapie "VT2" angewandt worden war, ein anschließender Übergang auf einen Wert oberhalb tf zur Anwendung der Therapie "VF", eine Verlangsamung in das Gebiet zwischen tt1 und tt3 jedoch zur Beibehaltung der Therapie "VT2" führt.
- Zeile f) schließlich zeigt, daß ausgehend von einer Rate oberhalb von tt3, bei der die Therapie VF angewandt wurde, eine Verlangsamung in das Gebiet zwischen tt1 und tt3 hinein zur Umschaltung auf die Therapie "VT2" führt.

Die praktische Realisierung der Funktionsblöcke der in den Figuren dargestellten Anordnungen ebenso wie die Wahl geeigneter Parameter zur Ausführung der einzelnen Therapien, etwa als spezifische Stimulationsimpulsfolgen (bzw. der Therapie "VF" als Defibrillationsschock), liegt im Rahmen fachmännischen Handelns und bedarf daher hier keiner genaueren Erläuterung. Es ist darauf hinzuweisen, daß auch eine Realisierung der Funktionselemente im Rahmen einer Mikroprozessorsteuerung - auch softwaremäßig - im Rahmen der in den Ansprüchen definierten Erfindung liegt.

## Patentansprüche

1. Therapiegerät, insbesondere implantierbarer Herzschrittmacher (1; 101), Kardioverter, kombinierter Herzschrittmacher/Kardioverter (301) oder Medikamentendosiergerät (201), mit mindestens einem Sensor (3; 103.1, 103.2; 203; 303) zum Erfassen einer bei Anwendung einer vorbestimmten Therapie im oder am Körper eines Patienten (P) meßbaren, insbesondere dessen körperlichen Zustand kennzeichnenden, Größe und zur Ausgabe eines entsprechenden Meßwertes, einer mindestens mittelbar mit dem Ausgang des Sensors verbundenen Auswertungs- und Steuereinrichtung (8; 108, 210; 310, 311) zur Auswertung des Meßwertes und zur Bestimmung einer Therapiesteuergröße in Abhängigkeit hiervon und einer mit dem Ausgang der Auswertungs- und Steuereinrichtung verbundenen Therapieeinrichtung (10; 110; 212; 311.3, 311.4, 312), die zur Realisierung verschiedener Therapien oder Therapiegrößen in Abhängigkeit vom Wert der Therapiesteuergröße ausgebildet ist.
**dadurch gekennzeichnet, daß**
zwischen den Ausgang des Sensors und den Eingang der Auswertungs- und Steuereinrichtung eine eingangsseitig mit dem Ausgang eines Schwankungswertgenerators (6, 7; 106, 107; 208, 209; 308) verbundene Verarbeitungseinheit (5; 105; 207; 307) geschaltet ist, in der unter Steuerung durch eine Zeitsteuereinheit (7.1; 107.1; 206; 306) dem Sensor-Meßwert zu mindestens einem Zeitpunkt mindestens ein Schwankungswert aufgeprägt wird derart, daß bei Auswertung des mit dem oder einem der Schwankungswert(e) korrigierten Meßwertes ein gegenüber der Auswertung des ursprünglichen Meßwertes und/oder des mit einem anderen Schwankungswert korrigierten Meßwertes veränderter Wert der Therapiesteuergröße erhalten wird.

2. Therapiegerät nach Anspruch 1, **dadurch gekennzeichnet, daß** dem Sensor (3; 103.2) ein Vergleichswertspeicher (14; 114) zugeordnet und eine eingangsseitig mit den Ausgängen des Sensors und des Vergleichswertspeichers und ausgangsseitig mit einer Schalteinheit (7; 107) verbundene Vergleichereinheit (13; 113) vorgesehen ist, in der der Sensor-Meßwert einem Vergleich mit mindestens einem gespeicherten Vergleichswert unterzogen wird und die im Ergebnis des Vergleiches ein Steuersignal an die Schalteinheit abgibt, welche in Abhängigkeit von diesem Steuersignal den Sensor-Meßwert entweder dem Eingang der mathematischen Verarbeitungseinheit (5; 105) oder unter Umgehung dieser direkt dem Eingang der Auswertungs- und Steuereinrichtung (8, 108) zuführt.

3. Therapiegerät nach Anspruch 1, **dadurch gekennzeichnet, daß** ein erster (103.1) und ein zweiter Sensor (103.2) vorgesehen sind, wobei dem Ausgang des ersten Sensors der Schwankungswertgenerator (106) zugeordnet ist derart, daß mit dem Schwankungswert korrigierte Meßwerte des ersten Sensors der Variation der Therapie bzw. Therapiegröße zugrundegelegt werden, während der Ausgang des zweiten Sensors mindestens mittelbar mit einem Eingang der Zeitsteuereinheit (107.1) verbunden ist derart, daß die Aufprägung der Schwankungsgröße auf die Meßwerte des ersten Sensors (103.1) in Abhängigkeit von den Meßwerten des zweiten Sensors (103.2) gesteuert, insbesondere wahlweise unterbunden, wird.

4. Therapiegerät nach Anspruch 3 , **dadurch gekennzeichnet, daß** der zweite Sensor (103.2) zur Erfassung einer von einer Organfunktion oder der Therapiegröße abhängigen, den körperlichen Zustand des Patienten kennzeichnenden Größe, insbesondere des Blutdrucks, ausgebildet ist.

5. Therapiegerät nach einem der vorangehenden Ansprüche, **dadurch gekennzeichnet, daß** der bzw. der erste Sensor (3; 103.1; 203; 303) zur Erfassung einer Aktivitätsgröße oder eine Organfunktion des Patienten (P) kennzeichnenden Größe oder zur Erfassung der Therapiegröße ausgebildet ist.

6. Therapiegerät nach einem der vorangehenden Ansprüche, **dadurch gekennzeichnet, daß** der Schwankungswertgenerator (106, 107; 208, 209; 308, 309) zur Ausgabe mindestens eines Inkrement- oder Dekrementwertes und die mathematische Verarbeitungseinheit als Additionsstufe (105; 205; 305) ausgebildet ist oder daß der Schwankungswertgenerator (6, 7) zur Ausgabe mindestens eines Korrekturfaktors und die mathematische Verarbeitungseinheit als Multiplikationsstufe (5) ausgebildet ist.

7. Therapiegerät nach einem der vorangehenden Ansprüche, **dadurch gekennzeichnet, daß** der Schwankungswertgenerator einen Schwankungswertspeicher (6; 106; 208; 308) für mehrere Schwankungswerte und wahlweise einen Zufallsgenerator zur Auswahl je eines der gespeicherten Schwankungswerte zur Aufprägung auf den Meßwert aufweist.

8. Therapiegerät nach einem der vorangehenden Ansprüche, **gekennzeichnet durch** die Ausbildung als implantierbarer Herzschrittmacher und/oder Kardioverter (301) derart, daß der bzw. der erste Sensor (303) **durch** eine intrakardiale Elektrode mit nachgeschaltetem Abfühlverstärker (304) für elektrische Aktivität des Herzens und eine Einrichtung (305) zur Bestimmung der Periode der elektrischen Herzaktivität als Meßwert gebildet und die Auswertungs- und Steuereinrichtung (311.1) zur Festlegung einer vorgegebenen Folge elektrischer Stimulationsimpulse und/oder eines Einzelimpulses und die Therapieeinrichtung (311.3) zur Erzeugung und Abgabe des/der elektrischen Stimulationsimpulse(s) als Therapie an das Herz ausgebildet sind.

9. Therapiegerät nach einem der Ansprüche 1 bis 7, **gekennzeichnet durch die** Ausbildung als implantierbarer Herzschrittmacher (101) derart, daß der bzw. der erste Sensor (103.1) zur Erfassung einer die körperliche Aktivität des Patienten repräsentierenden Größe als Meßwert, die Auswertungs- und Steuereinrichtung (108) zur Festlegung der Rate elektrischer Stimulationsimpulse und die Therapieeinrichtung (110) zur Erzeugung und Abgabe der elektrischen Stimulationsimpulse mit der festgelegten Rate als Therapiegröße an das Herz ausgebildet sind.

10. Therapiegerät nach einem der Ansprüche 1 bis 7, **gekennzeichnet durch** die Ausbildung als, insbesondere implantierbares, Medikamentendosiergerät (201) derart, daß der bzw. der erste Sensor (203) zur Erfassung des Pegels eines Wirkstoffes oder einer davon abhängigen Größe im Körper des Patienten (P) als Meßwert, die Auswertungs- und Steuereinrichtung (210) zur Festlegung einer Medikamentendosis pro Zeiteinheit und die Therapieeinrichtung (212) zur Abgabe der festgelegten Dosis pro Zeiteinheit an den Körper ausgebildet sind.

11. Therapiegerät nach Anspruch 10, **gekennzeichnet durch** die Ausbildung des bzw. des ersten Sensors als intrakardiale Elektrode (203) mit nachgeschaltetem Abfühlverstärker (204) für elektrische Aktivität des Herzens mit zugeordneter Einrichtung zur Bestimmung der Periode der elektrischen Herzaktivität als Meßwert.

12. Therapiegerät nach einem der vorangehenden Ansprüche, **dadurch gekennzeichnet, daß** die Auswertungs- und Steuereinrichtung (310, 311)
- einen Bereichsgrenzenspeicher (315) zur Speicherung mindestens zweier verschiedener Grenzen zwischen zwei Wertebereichen der Meßgröße und damit mindestens einer Überlappungszone zwischen den Wertebereichen,
- eine über einen Eingang mindestens mittelbar mit dem Sensor (303) und über einen weiteren Eingang mit dem Bereichsgrenzenspeicher (315) verbundene erste Vergleichereinheit (310.1) zur Zuordnung des Meßwertes der Größe zu einem der Wertebereiche,
- einen Therapiespeicher (311.2) mit mindestens zwei separat adressierbaren Speicherbereichen zur Speicherung mindestens zweier verschiedener Werte der Therapiesteuergröße in Zuordnung zu den Werten der Meßgröße innerhalb einer Überlappungszone jeweils zusammen mit einer eine Meßgrößenoder Therapie-Vorgeschichte repräsentierenden vorgegebenen Bedingungsgröße und weiteren Speicherbereichen zur Speicherung jeweils eines Wertes der Therapiesteuergröße in Zuordnung zu einem Wert der Meßgröße außerhalb einer Überlappungszone,
- einen Vorgeschichte-Speicher (317) zur Speicherung der Bereichszuordnung des jeweils vorhergehenden Wertes der Meßgröße oder der Therapiesteuergröße einer vorher angewandten Therapie als Bedingungsgröße und
- Vorgeschichte-Auswertungsmittel (318) zur Auswertung des gespeicherten Wertes mit den vorgegebenen Werten der Bedingungsgröße und zur Ausgabe von das Auswertungsergebnis ausdrückenden Adreßdaten an den Therapiespeicher zum Auslesen genau eines Wertes der Therapiesteuergröße aufweist.

13. Therapiegerät nach Anspruch 12 und einem der Ansprüche 1-9, **gekennzeichnet durch** die Ausführung als implantierbarer Bedarfsschrittmacher (311) mit einem Bereichsgrenzenspeicher (315), der zur Speicherung mindestens einer Grenze zwischen einem Normalund einem Bradykardie-Ratenbereich sowie zweier Grenzen und somit einer Überlappungszone zwischen dem Normal- und einem Tachykardie-Ratenbereich, sowie einem Therapiespeicher (311.2), der zur Speicherung mindestens einer Bradykardieund einer Tachykardie-Therapie ausgebildet ist.

14. Therapiegerät nach Anspruch 12 und einem der Ansprüche 1-9, **gekennzeichnet durch** die Ausführung als implantierbares Antitachykardiegerät, insbesondere Antitachykardie-Schrittmacher/Defibrillator (301), mit einem Bereichsgrenzenspeicher (315), der zur Speicherung mindestens einer Grenze zwischen einem Normalund einem Tachykardie-Ratenbereich sowie mindestens zweier Grenzen und somit einer Überlappungszone zwischen dem oder einem Tachykardie-Ratenbereich und einem Fibrillationsbereich ausgebildet ist.

## Claims

1. Therapy device, in particular implantable cardiac pacemaker (1; 101), cardioverter, combined cardiac pacemaker/cardioverter (301) or medication dosing device (201), comprising at least one sensor (3; 103.1, 103.2; 203; 303) for detecting a variable which is measurable in or on the body of a patient (P) during the application of a predetermined therapy and in particular characterising his physical state and for outputting a corresponding measured value, an evaluation and control device (8; 108, 210; 310, 311) connected at least indirectly to the output of the sensor for evaluating the measured value and determining a therapy control variable as a function thereof, and a therapy device (10; 110; 212; 311.3, 311.4, 312) connected to the output of the evaluation and control device and designed to produce various therapies or therapy variables as a function of the value of the therapy control variable, **characterised in that** connected between the output of the sensor and the input of the evaluation and control device is a processing unit (5; 105; 207; 307) which is connected on the input side to the output of a fluctuation value generator (6, 7; 106, 107; 208, 209; 308), in which processing unit at least one fluctuation value is impressed on the sensor measured value at at least one instant with control by a time control unit (7.1; 107.1; 206; 306) in such a way that during evaluation of the measured value corrected by the, or one of the fluctuation value(s), a value of the therapy control variable which is changed relative to the evaluation of the original measured value and/or of the measured value corrected by another fluctuation value is obtained.

2. Therapy device according to claim 1, **characterised in that** a comparison value memory (14; 114) is associated with the sensor (3; 103.2) and a comparison unit (13; 113) connected on the input side to the outputs of the sensor and the comparison value memory and on the output side to a switching unit (7; 107) is provided, in which the sensor measured value is subjected to a comparison with at least one stored comparison value and which, as a result of the comparison, emits a control signal to the switching unit which as a function of this control signal supplies the sensor measured value either to the input of the mathematical processing unit (5; 105) or directly to the input of the evaluation and control device (8, 108) by bypassing the processing unit.

3. Therapy device according to claim 1, **characterised in that** a first (103.1) and a second sensor (103.2) are provided, wherein the fluctuation value generator (106) is associated with the output of the first sensor in such a way that measured values of the first sensor corrected by the fluctuation value form the basis for the variation in the therapy or therapy variable, while the output of the second sensor is at least indirectly connected to an input of the time control unit (107.1) in such a way that the impression of the fluctuation variable onto the measured value of the first sensor (103.1) is controlled, in particular optionally stopped, as a function of the measured values of the second sensor (103.2).

4. Therapy device according to claim 3, **characterised in that** the second sensor (103.2) is designed to detect a variable, in particular of the blood pressure, dependent on an organ function or the therapy variable and characterising the physical state of the patient.

5. Therapy device according to any one of the preceding claims, **characterised in that** the, or the first sensor (3; 103.1; 203; 303) is designed to detect an activity variable or a variable characterising an organ function of the patient (P) or to detect the therapy variable.

6. Therapy device according to any one of the preceding claims, **characterised in that** the fluctuation value generator (106, 107; 208, 209; 308, 309) is designed to output at least one increment or decrement value and the mathematical processing unit is designed as an addition stage (105; 205; 305) or **in that** the fluctuation value generator (6, 7) is designed to output at least one correction factor and the mathematical processing unit is designed as a multiplication stage (5).

7. Therapy device according to any one of the preceding claims, **characterised in that** the fluctuation value generator has a fluctuation value memory (6; 106; 208; 308) for a plurality of fluctuation values and optionally a random generator for selecting one of the respective stored fluctuation values for impressing on the measured value.

8. Therapy device according to any one of the preceding claims, **characterised by** the design as an implantable cardiac pacemaker and/or cardioverter (301) in such a way that the, or the first sensor (303) is formed by an intracardial electrode comprising a downstream sensor amplifier (304) for electrical activity of the heart and a device (305) for determining the period of the electrical cardiac activity as a measured value and the evaluation and control device (311.1) is designed to establish a predetermined sequence of electrical stimulation pulses and/or of an individual pulse and the therapy device (311.3) is designed to generate and output the electrical stimulation pulse(s) to the heart as therapy.

9. Therapy device according to any one of claims 1 to 7, **characterised by** the design as an implantable cardiac pacemaker (101), in that the, or the first sensor (103.1) is designed to detect a variable representing the physical activity of the patient, as a measured value, and the evaluation and control device (108) are designed to establish the rate of the electrical stimulation pulses, and the therapy device (110) is designed to generate and output the electrical stimulation pulses at the established rate to the heart as a therapy variable.

10. Therapy device according to any one of claims 1 to 7, **characterised by** the design as, an in particular implantable medication dosing device (201), in such a way that the, or the first sensor (203) is designed to detect the level of an active ingredient or a variable dependent thereon in the body of the patient (P) as a measured value, and the evaluation and control device (210) are designed to establish a medication dose per time unit, and the therapy device (212) is designed to deliver the established dose per time unit to the body.

11. Therapy device according to claim 10, **characterised by** the design of the, or the first sensor as an intracardial electrode (203) with downstream sensor amplifier (204) for the electrical activity of the heart comprising an associated device for determining the period of electrical cardiac activity, as the measured variable.

12. Therapy device according to any one of the preceding claims, **characterised in that** the evaluation and control device (310, 311) has
- a range-limit memory (315) for storing at least two different limits between two value ranges of the measured variable and therefore at least one overlapping zone between the value ranges,
- a first comparator unit (301.1) at least indirectly connected to the sensor (303) via an input and to the range-limit memory (315) via a further input for association of the measured value of the variable with one of the value ranges,
- a therapy memory (311.2) with at least two separately-addressable memory regions for storing at least two different values of the therapy control variable in association with the values of the measured variable within an overlapping zone, in each case together with a predetermined conditional variable representing a past history of the measured variable or therapy and with further memory regions for storing, in each case, a value of the therapy control variable in association with a value of the measured variable outside an overlapping zone,
- a past history memory (317) for storing the range association of the respective previous value of the measured variable or the therapy control variable of a previously used therapy as the conditional variable and
- a past history evaluation means (318) for evaluating the stored value with the predetermined values of the conditional variable and for outputting the address data expressing the evaluation result to the therapy memory to precisely read out a value of the therapy control variable.

13. Therapy device according to claim 12 and any one of claims 1 to 9, **characterised by** the design as an implantable demand pacemaker (311) comprising a range limit memory (315) designed to store at least one limit between a normal rate range and bradycardic rate range and two limits and therefore an overlapping zone between the normal rate range and a tachycardiac rate range, and a therapy memory (311.2) designed to store at least one bradycardic therapy and one tachycardiac therapy.

14. Therapy device according to claim 12 and any one claims 1 to 9, **characterised by** the design as an implantable anti-tachycardiac device, in particular an anti-tachycardiac pacemaker/defibrillator (301), comprising a range limit memory (315) which is designed to store at least one limit between a normal rate range and a tachycardiac rate range and at least two limits and therefore an overlapping zone between the, or one tachycardiac rate range and a fibrillation range.

## Revendications

1. Appareil de thérapie, notamment stimulateur cardiaque implantable (1; 101), dispositif de cardioversion, stimulateur cardiaque/dispositif de cardioversion combiné (301) ou appareil de dosage de médicament (201), comportant au moins un capteur (3; 303.1, 303.2; 203; 303) pour détecter une grandeur mesurable lors de l'application d'une thérapie prédéterminée dans ou sur le corps d'un patient (P), et caractérisant notamment son état corporel, et pour délivrer une valeur de mesure correspondante, un dispositif d'évaluation et de commande (8; 108, 210; 310, 311) relié au moins indirectement à la sortie du capteur et servant à évaluer la valeur de mesure et à déterminer une grandeur de commande de thérapie en fonction de cette grandeur et un dispositif de thérapie (10; 110; 212; 311.3, 311.4, 312) relié à la sortie du dispositif d'évaluation et de commande et qui est agencé et conçu pour appliquer différentes thérapies ou obtenir différentes grandeurs de thérapie en fonction de la valeur de la grandeur de commande de thérapie,
**caractérisé en ce que**
entre la sortie du capteur et l'entrée du dispositif d'évaluation et de commande est branchée une unité de traitement (5; 105; 207; 307), qui est reliée à la sortie d'un générateur de valeurs de variation (6, 7; 106, 107; 208, 209; 308) et dans laquelle au moins une valeur de variation est ajoutée au moins à un instant à la valeur de mesure du capteur, sous la commande d'une unité de commande temporelle (7.1; 107.1; 206; 306), de telle sorte que lors de l'évaluation de la valeur de mesure corrigée avec la ou une des valeurs de variation, on obtient une valeur de la grandeur de commande de thérapie, modifiée par rapport à l'évaluation de la valeur de mesure initiale et/ou de la valeur de mesure corrigée avec une autre valeur de variation.

2. Appareil de thérapie selon la revendication 1, **caractérisé en ce qu'**une mémoire de valeurs de comparaison (14; 114) est associée au capteur (3; 103.2) et qu'il est prévu une unité de comparaison (13; 113), qui est reliée côté entrée aux sorties du capteur et de la mémoire de valeurs de comparaison et côté sortie à une unité de commutation (7; 107) et dans laquelle la valeur de mesure du capteur est comparée à au moins une valeur de comparaison mémorisée et qui, en tant que résultat de la comparaison, délivre un signal de commande à l'unité de commutation qui, en fonction de ce signal de commande, envoie la valeur de mesure du capteur soit à l'entrée de l'unité de traitement mathématique (5; 105), soit directement, en contournant cette unité, à l'entrée du dispositif d'évaluation et de commande (8, 108).

3. Appareil de thérapie selon la revendication 1, **caractérisé en ce qu'**il est prévu un premier capteur (103.1) et un second capteur (103.2), le générateur (106) de valeurs de variation étant associé à la sortie du premier capteur de telle sorte que des valeurs de mesure, corrigées avec la valeur de variation, du premier capteur sont prises comme base pour la modification de la thérapie ou de la grandeur de thérapie, tandis que la sortie du second capteur est reliée au moins indirectement à une entrée de l'unité de commande temporelle (107.1) de telle sorte que l'addition de la grandeur de variation aux valeurs de mesure du premier capteur (103.1) est commandée en fonction de la valeur de mesure du second capteur (103.2), notamment est éliminée au choix.

4. Appareil de thérapie selon la revendication 3, **caractérisé en ce que** le second capteur (103.2) est agencé de manière à détecter une grandeur qui dépend d'une fonction d'un organe ou de la grandeur de thérapie et caractérise l'état corporel du patient, notamment la pression sanguine.

5. Appareil de thérapie selon l'une des revendications précédentes, **caractérisé en ce que** le capteur ou le premier capteur (3; 103.1; 203; 303) est agencé de manière à détecter une grandeur d'activité ou une grandeur caractérisant une fonction d'un organe du patient (P) ou est agencé pour détecter la grandeur de thérapie.

6. Appareil de thérapie selon l'une des revendications précédente, **caractérisé en ce que** le générateur de valeurs de variation (106, 107; 208, 209; 308, 309) est agencé de manière à délivrer au moins une valeur d'incrément ou une valeur de décrément et l'unité de traitement mathématique est agencée sous la forme d'un étage d'addition (105; 205; 305), ou que le générateur de valeurs de variation (6, 7) est agencé de manière à délivrer au moins un facteur de correction et l'unité de traitement mathématique est agencée sous la forme d'un étage de multiplication (5).

7. Appareil de thérapie selon l'une des revendications précédentes, **caractérisé en ce que** le générateur de valeurs de variation comporte une mémoire de valeurs de variation (6; 106; 208; 308) pour plusieurs valeurs de variation et au choix un générateur de nombres aléatoire pour sélectionner respectivement l'une des valeurs de variation pour l'ajouter à la valeur de mesure.

8. Appareil de thérapie selon l'une des revendications précédentes, **caractérisé par** sa réalisation sous la forme d'un stimulateur cardiaque et/ou d'un dispositif de cardioversion implantable (301) de telle sorte que le capteur ou le premier capteur (303) est formé par une électrode intracardiaque en aval de laquelle est branché un amplificateur de détection (304) servant à détecter l'activité électrique du coeur, et par un dispositif (305) pour déterminer la période de l'activité cardiaque électrique en tant que valeur de mesure, et que le dispositif d'évaluation et de commande (311.1) est agencé de manière à fixer une séquence prédéterminée d'impulsions de stimulation électrique et/ou d'une impulsion individuelle et le dispositif de thérapie (311.3) est agencé de manière produire et délivrer la ou les impulsions de stimulation électrique en tant que thérapie appliquée au coeur.

9. Appareil de thérapie selon l'une des revendications 1 à 7, **caractérisé par** son agencement en tant que stimulateur cardiaque implantable (101) de telle sorte que le capteur ou le premier capteur (103.1) est agencé de manière à détecter en tant que valeur de mesure une grandeur représentant l'activité corporelle du patient, que le dispositif d'évaluation et de commande (108) est agencé de manière à fixer la cadence d'impulsions de stimulation électrique et que le dispositif de thérapie (110) est agencé de manière à produire et délivrer les impulsions de stimulation électrique possédant la cadence fixée, en tant que grandeur de thérapie au coeur.

10. Appareil de thérapie selon l'une des revendications 1 à 7, **caractérisé par** son agencement en tant qu'appareil de dosage de médicament (201), notamment implantable de telle sorte que le capteur ou le premier capteur (203) est agencé pour détecter en tant que valeur de mesure le niveau d'une substance active ou d'une grandeur, qui en dépend, dans le corps du patient (P), que le dispositif d'évaluation et de commande (210) est agencé de manière à fixer une dose de médicament par unité de temps et que le dispositif thérapie (212) est agencé de manière à délivrer la dose fixée par unité de temps au corps.

11. Appareil de thérapie selon la revendication 10, **caractérisé par le fait que** le capteur ou le premier capteur est agencé sous la forme d'une électrode intracardiaque (203) en aval de laquelle est branché un amplificateur de détection (204) servant à détecter une activité électrique du coeur et auquel est associé un dispositif pour déterminer la période de l'activité cardiaque électrique en tant que valeur de mesure.

12. Appareil de thérapie selon l'une des revendications précédentes, **caractérisé en ce que** le dispositif d'évaluation et de commande (310, 311) comporte
- une mémoire (304) de limites de gammes pour mémoriser au moins deux limites différentes entre deux gammes de valeurs de la grandeur de mesure et par conséquent au moins une zone de chevauchement entre les gammes de valeurs,
- une première unité de comparaison (310.1), qui est reliée par l'intermédiaire d'une entrée au moins indirectement au capteur (303) et par l'intermédiaire d'une autre entrée à la mémoire (304) de limites de gammes et sert à associer la valeur de mesure de la grandeur à l'une des gammes de valeurs,
- une mémoire de thérapie (311.2) comportant au moins deux zones de mémoire adressables séparément et servant à mémoriser au moins deux valeurs différentes de la grandeur de commande de thérapie en association avec les valeurs de la grandeur de mesure à l'intérieur d'une zone de chevauchement, respectivement conjointement avec une grandeur conditionnelle prédéterminée, qui représente un historique de la grandeur de mesure ou de la thérapie, et d'autres zones de mémoire servant à mémoriser respectivement une valeur de la grandeur de commande de thérapie en association avec une valeur de la grandeur de mesure à l'extérieur d'une zone de chevauchement,
- une mémoire d'historique (317) pour mémoriser l'association de gammes de la valeur respectivement précédente de la grandeur de mesure ou de la grandeur de commande de thérapie à une thérapie utilisée antérieurement, en tant que grandeur conditionnelle, et
- des moyens (318) d'évaluation d'historique pour évaluer la valeur mémorisée avec les valeurs prédéterminées de la grandeur conditionnelle et pour délivrer le résultat des données d'adresses exprimant le résultat de l'évaluation à la mémoire de thérapie pour permettre la lecture précisément d'une valeur de la grandeur de commande de thérapie.

13. Appareil de thérapie selon la revendication 12 et l'une des revendications 1 à 9, **caractérisé par** sa réalisation sous la forme d'un stimulateur cardiaque de secours implantable (311) comportant une mémoire (304) de limite de gammes, qui est agencée pour mémoriser au moins une limite entre une gamme de rythme normaux et une gamme de rythmes de bradycardie, ainsi que deux limites et par conséquent une zone de chevauchement entre une gamme de rythmes normaux et une gamme de rythmes de tachycardie, ainsi qu'une mémoire de thérapie (311.2), qui est agencée pour la mémorisation d'au moins une thérapie de bradycardie et une thérapie de tachycardie.

14. Appareil de thérapie selon la revendication 12 et l'une des revendications 1 à 9, **caractérisé par** son agencement sous la forme d'un appareil implantable d'antitachycardie, notamment sous la forme d'un stimulateur cardiaque/défibrillateur d'anti-tachycardie (301), comportant une mémoire (304) de limites de gammes, qui est agencée de manière à mémoriser au moins une limite entre une gamme de rythmes normaux et une gamme de rythmes de tachycardie ainsi qu'au moins deux limites et par conséquent une zone de chevauchement entre la ou une gamme de rythmes de tachycardie et une gamme de fibrillation.
